# EUROPEAN PATENT SPECIFICATION

(11) **EP 3 934 730 B1**
(45) Date of publication and mention of the grant of the patent: **16.10.2024**
(21) Application number: 20766827.8
(22) Date of filing: 06.03.2020
(51) Int. Cl.: A61M 27/00, A61B 17/3209, A61M 3/02

(54) **CATHETER DEVICE INCLUDING A CONNECTOR**
KATHETERVORRICHTUNG MIT EINEM VERBINDER
DISPOSITIF CATHÉTER COMPRENANT UN CONNECTEUR

(30) Priority: 06.03.2019 US 201916293932
(43) Date of publication of application: 12.01.2022
(73) Proprietor: EM Device Lab, Inc., Austin, TX 78731 (US)
(72) Inventor: GORN, Michael, Austin, Texas 78731 (US); MCGREGOR, Gary, Pflugerville, Texas 78660 (US)
(74) Representative: Samson & Partner Patentanwälte mbB
(86) International application number: PCT/US2020/021338
(87) International publication number: WO 2020/181172

(56) References cited:
- CA-A1- 3 038 516
- US-A- 5 354 282
- US-A1- 2017 224 967
- US-A1- 2017 224 967
- US-A1- 2020 001 057
- US-B1- 6 290 691
- US-B2- 6 893 424
- US-B2- 9 518 667

## Description

### Field

The present disclosure is generally related to catheter devices, and more particularly to catheter devices that include a connector and methods thereof. In some embodiments, the catheter devices may be configured to treat cutaneous and oral abscesses.

### Background

Surgical drains are used in a wide variety of different surgical procedures, for example, to drain fluid from a surgical area. Some examples where such drains are used may include plastic surgery, breast surgery (to prevent collection of blood, lymph fluid, or both), orthopedic procedures, chest drainage, infected cysts, pancreatic surgery (to drain secretions), biliary surgery, thyroid surgery, neurosurgery (to remediate risk of intracranial pressure), urinary catheters, nasogastric tubes, and other procedures.

One class of such surgical drains may be used on patients with cutaneous and oral abscesses, or collections of pus, hematomas, seromas or any other fluids requiring drainage. For example, abscesses can form anywhere in the body, from a superficial skin (subcutaneous) abscess to deep abscesses in muscle, organs, or body cavities. Treatment of such abscesses typically involves draining the accumulated fluid (such as pus) to resolve the infection or cause of the abscess and to facilitate recovery. One class of such surgical drains may require the patient or a care giver to adjust the drain to reopen clogged drainage paths to facilitate drainage.

The approach used to drain the accumulated fluid may depend on the size and location of the abscess. For subcutaneous abscesses, treatment typically includes creating an incision through the layers of the skin into the abscess cavity using a scalpel, expressing fluid (e.g., pus) from the abscess, and optionally using a hemostat to explore the wound and to break up pockets or localized areas of hardened pus. In some instances, packing material (such as a strip of gauze) may be inserted into the abscess cavity to prevent skin closure and re-accumulation of fluid in the abscess and to enable continued drainage. In other instances, a drainage catheter may be inserted through the incision and into the abscess to facilitate drainage and optionally irrigation of the abscess cavity.

The approach to draining abscesses in the oral cavity typically includes incision through the mucosa to the abscess cavity using a scalpel, expressing fluid (e.g., pus) from the abscess, and optionally using a hemostat to explore the wound and to break up pockets or localized areas of hardened pus. In most instances, a small drain is sutured into the cavity of the abscess cavity to mucosal closure and re-accumulation of fluid in the abscess and to enable continued drainage.

CA 3 038 516 relates to drainage catheter systems including a hub and discloses subject-matter according to the preamble of claim 1.

### Summary

The present invention is defined in the appended independent claim. The dependent claims relate to preferred embodiments. No surgical methods are claimed per-se.

In some embodiments, a drainage device may include a catheter including a proximal end and a distal end. The drainage device may further include a connector coupled to the proximal end of the catheter. The connector may include a body portion including a port sized to receive a tip of a syringe and including a lumen extending from the port to the proximal end of the catheter. The body portion may further include a hinged element configured to secure the distal end of the catheter against the body portion and to clamp the distal end of the catheter closed.

In some embodiments, a drainage device may include a catheter having a proximal end and a distal end and may include a connector coupled to the proximal end of the catheter and including a hinged fastener. The hinged fastener may be configured to close over a portion of the catheter near the distal end to form a loop and to clamp the distal end closed to prevent fluid flow through the distal end.

In some embodiments, a catheter device may include a catheter including a proximal end and a distal end. The catheter device may further include a connector coupled to the proximal end of the catheter. The connector may include a base having a recess configured to receive the distal end of the catheter and may include a hinged portion configured to couple to the base to clamp the catheter within the recess.

In other embodiments, a catheter device may include a catheter including a proximal end and a distal end and defining a lumen extending from the proximal end to the distal end. The catheter may include a plurality of openings extending from the lumen through a wall of the catheter. The catheter device may further include a connector coupled to the proximal end of the catheter. The connector may include a clamp configured to close over a portion of the catheter to compress the lumen to prevent fluid flow.

In still other embodiments, a catheter device may include a catheter including a proximal end and a distal end. The catheter may define a lumen extending from the proximal end to the distal end. The catheter device can further include a connector including a catheter-engagement element coupled to the proximal end. The connector may include a fluid port and may define a fluid passage extending from the fluid port to the lumen of the catheter through the catheter-engagement element. The connector may further include a base and a hinged element configured to couple to the base to clamp a portion of the catheter to compress the lumen to prevent fluid flow.

### Brief Description Of The Drawings

FIG. 1 depicts a diagram of a catheter device including a connector, in accordance with certain embodiments of the present disclosure.
FIG. 2 depicts a portion of the catheter device of FIG. 1 including the connector coupled to a syringe, in accordance with certain embodiments of the present disclosure.
FIG. 3 depicts a side view of the connector, in accordance with certain embodiments of the present disclosure.
FIG. 4 depicts a top view of a needle of the catheter device of FIG. 1, in accordance with certain embodiments of the present disclosure.
FIG. 5 depicts a portion of the catheter including a helical arrangement of openings, in accordance with certain embodiments of the present disclosure.
FIG. 6 depicts a diagram of an embodiment of the catheter device configured to drain an abscess, in accordance with certain embodiments of the present disclosure.
FIG. 7 depicts a flow diagram of a method of producing a catheter device, in accordance with certain embodiments of the present disclosure.

In the following discussion, the same reference numbers are used in the various embodiments to indicate the same or similar elements.

### Detailed Description Of Illustrative Embodiments

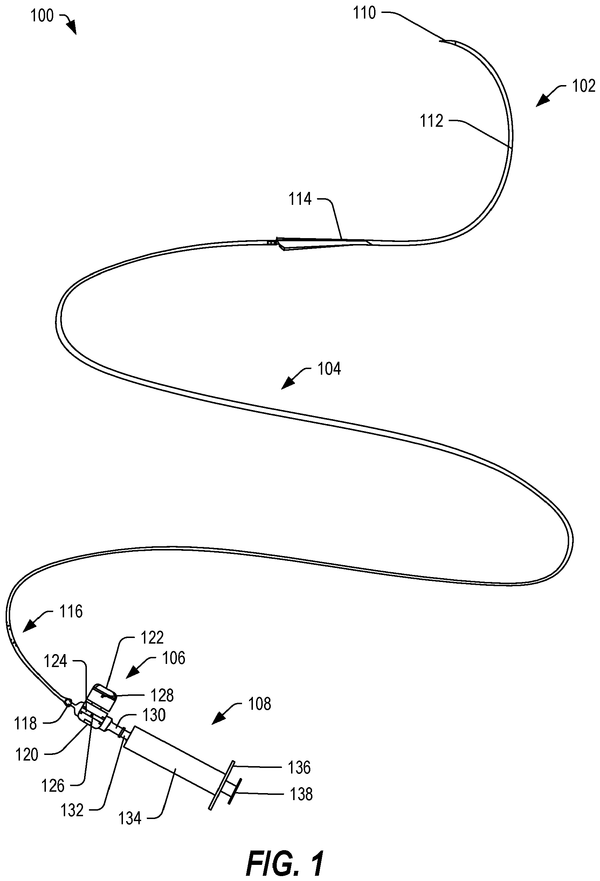

FIG. 1 depicts a diagram of a catheter device 100 including a connector 106, in accordance with certain embodiments of the present disclosure. The catheter device 100 may include a needle 102, a catheter 104 coupled to the needle 102, and a connector 106 coupled to the catheter 104. In the illustrated example, the catheter 104 may define a lumen extending from a proximal end coupled to the needle 102 to a distal end coupled to the connector 106. The connector 106 may be releasably coupled to a syringe 108.

The needle 102 may include a point or tip 110 configured to puncture a surface or a plurality of layers, such as layers of skin. The needle 102 may further include a body portion 112 extending between the point 110 and a cutting blade 114. The body portion 112 may be curved according to a selected radius. The cutting blade 114 may be substantially triangle-shaped and may include a cutting edge along at least one side. In some embodiments, the point or tip 110 may puncture the surface and advance through underlying layers along a curved path defined by the curvature of the needle 102. The cutting blade 114 may trail the point or tip 110 along the curved path and the cutting edges of the cutting blade 114 may operate to widen the opening created by the point or tip 110. Further, trailing edges of the cutting blade 114 may be blunt or rounded such that reversing the direction of the force applied to the needle 102 may cause the blunt or rounded trailing edges to break up pockets or localized-areas of hardened pus.

The catheter 104 may include a long, flexible tube defining a lumen. The catheter 104 may be configured to allow fluid received from the connector 106 to flow through the lumen. Further, the catheter 104 may include a plurality of openings or holes, generally indicated at 116, which may be arranged in a spiral or helical pattern along a length of a portion of the catheter 104. The openings or holes 116 extend from the lumen within the catheter 104 through the exterior surface of the catheter 104 to allow fluid to flow from the lumen through the openings or holes 116.

The connector 106 may include a catheter-engagement element 118 configured to fit within the lumen of the catheter 104 and to secure the catheter 104 to the connector 106. The connector 106 may further include a base portion including clip 120 configured to engage and secure a hinged portion 122. The base portion may further include a recess or groove 124 sized to receive a distal end of the catheter 104. The recess or groove 124 may further include an opening or further recess 126 sized to receive a corresponding post 128 of the hinged portion 122. The connector 106 may also include a port 130 including an opening sized to receive a tip 132 of the syringe 108. The connector 106 may define a fluid passage extending from the fluid port 130 through the connector and to the lumen of the catheter 104 through the catheter-engagement element 118.

The syringe 108 may further include a barrel portion 134 coupled to the tip 132 and defining an enclosure. The barrel portion 134 may further include a flange 136. The syringe 108 may further include a plunger 138 sized to fit within the enclosure and configured to push fluid from the enclosure, through an opening in the tip 132, through the connector 106 and into the lumen of the catheter 104. Other embodiments are also possible.

In some embodiments, the needle 102 is inserted and pulled through one or more layers by a physician, drawing the catheter 104 through the layers. The physician may place a distal end of the catheter 104 into the recess 124 and may close the hinged portion 122, clamping the hinged portion 122 against the base portion and securing the post 128 within the recess 126 to close and seal the lumen of the catheter 104 between the post 128 of the hinged portion 120 and the opening or further recess 128 of the recess 124. By clamping down on the catheter 104, the lumen of the catheter 104 is closed off to fluid flow through the connector 106.

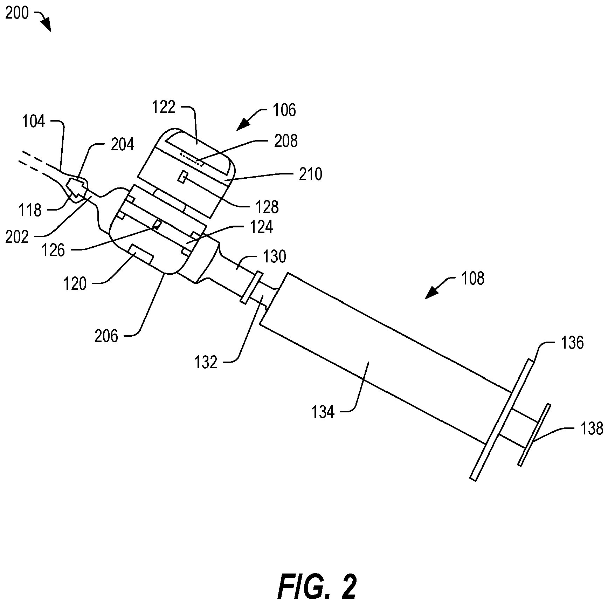

FIG. 2 depicts a portion 200 of the catheter device 100 of FIG. 1 including the connector 106 coupled to a syringe 108, in accordance with certain embodiments of the present disclosure. The connector 106 may include a catheter-engagement element 118 including a lumen configured to engage the lumen of the catheter 104. In this example, the catheter-engagement element 118 may include a polygonal shape including a neck portion 202 and a head portion 204. The distal end of the catheter 104 may stretch over the head portion 204 and tighten about the neck portion 202, securing the catheter 104 to the connector 106.

The connector 106 may further include a base portion 206, which defines a plurality of features configured to engage the hinged portion 122 and secure the catheter 104. The base portion 206 may include a latch or clip feature 120 configured to engage a corresponding feature or recess 208 formed beneath a reinforcing rib 210 on the hinged portion 122. The base portion 206 further includes the groove or recess 124 including an opening or notch 126 sized to receive the post 128 of the hinged portion 122. In operation, a portion of the catheter 104 may be placed into the groove or recess 124 and the hinged portion 122 may be closed, latching the hinged portion to the base portion 206 via the latch or clip feature 120 and the recess 208. Further, the post 128 may push into the notch 126 clamping the catheter 104 and compressing the lumen of the catheter 104 to close the catheter 104 to fluid flow.

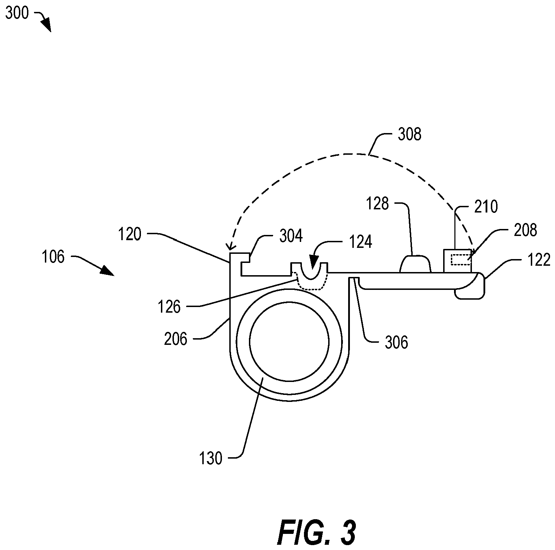

FIG. 3 depicts a side view 300 of the connector 106, in accordance with certain embodiments of the present disclosure. The connector 106 includes the port 130 to receive the tip of the syringe. Further, the connector 106 includes the base 206 coupled to a hinged portion 122 by a hinge 306. The base 206 includes a clip 120 including an extension 304 configured to engage a recess 208 in a reinforcing rib 210. Further, the hinged portion 122 includes a post 128 configured to fit within the opening or notch 126.

As shown in the illustrated example, the hinged portion 122 opens and closes along a path indicated by the dashed arrow 308. The extension 304 of the clip 120 may snap into the recess 208. Further, the post 128 may extend into the recess 124 and into the opening or notch 126 to clamp the catheter 104 within the recess 124. Other embodiments are also possible.

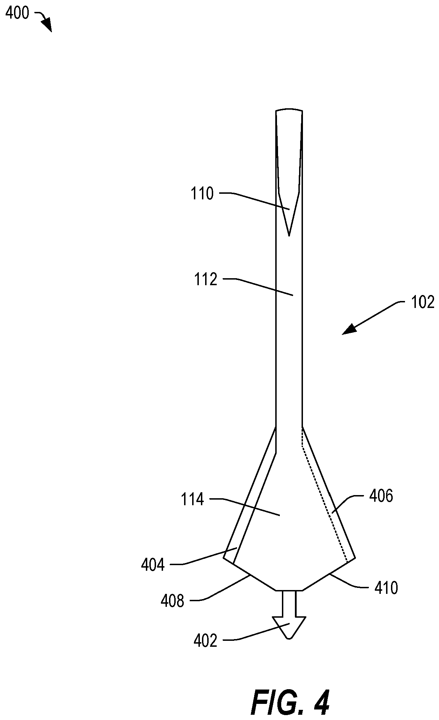

FIG. 4 depicts a top view 400 of the needle 102 of the catheter device 100 of FIG. 1, in accordance with certain embodiments of the present disclosure. The needle 102 includes the tip 110, the body portion 112, and the cutting blade 114. In this example, the cutting blade 114 may be substantially planar, while the tip 110 and the body portion 112 may define a curvature. The cutting blade 114 may extend outward from the body portion 112, forming wings or edges 404 and 406, which may be sharpened to facilitate widening of the opening formed by the tip 110 and the body 112 of the needle 102. In this example, one side of each of the edges 404 and 406 is sharpened. Trailing edges 408 and 410 may be blunted or rounded. The practitioner may manipulate the needle 102 to use the trailing edges to break up pockets within an abscess, for example.

A catheter-coupling element 402 may extend from an end of the needle 102 adjacent to the trailing edges 408 and 410. The element 402 may include an arrow-shaped or other-shaped feature 402 that can be pushed into the lumen of the catheter 104, such that the catheter 104 expands around the arrowhead of the element 402 and narrows to secure the catheter 104 to the needle 102.

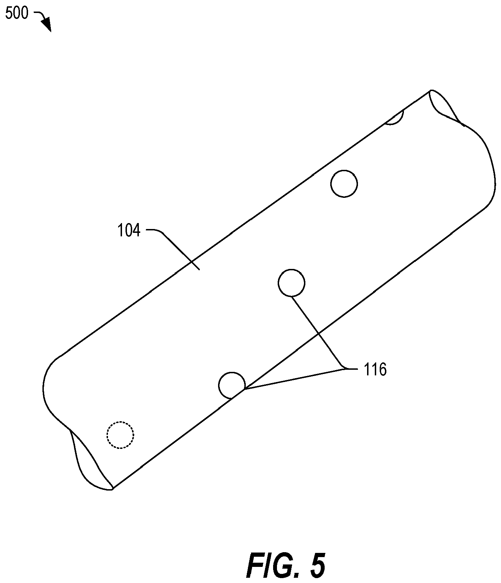

FIG. 5 depicts a portion 500 of the catheter 104 including a helical arrangement of openings 116, in accordance with certain embodiments of the present disclosure. As shown, openings 116 are provided in the catheter 104, which openings 116 extend from the lumen through the wall of the catheter 104 to provide drainage holes through which saline, antibiotics, other fluids, or any combination thereof may be delivered. In an example, the openings 116 may be positioned within an abscess by a practitioner, and the practitioner may press the plunger of a syringe coupled to the connector 106 to deliver fluid to the abscess via the lumen of the catheter 106 and through the openings 116.

In some embodiments, the arrangement of openings 116 may be helical, which allows for a substantially even distribution of fluid from the catheter 104, even if the catheter 104 is twisted. Other embodiments are also possible.

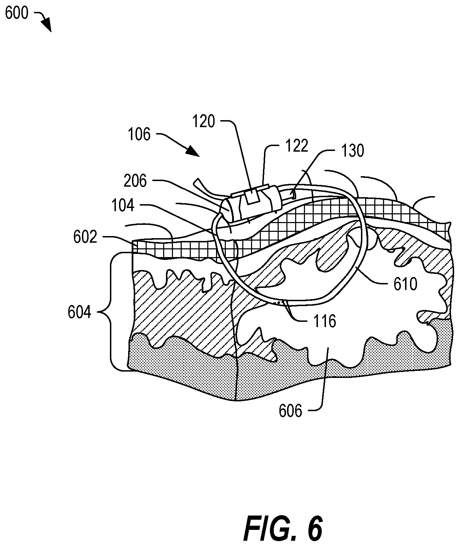

FIG. 6 depicts a diagram 600 of an embodiment of the catheter device 100 of FIG. 1 configured to drain an abscess, in accordance with certain embodiments of the present disclosure. The catheter device 106 includes a base 206 including a clip 120 configured to couple to the hinged portion 122 to clamp the distal end of the catheter 104 after the catheter 104 has been drawn into position within the abscess 606 by the needle 102. The catheter device 106 is coupled to a proximal end of the catheter 104 and is configured to provide a fluid conduit from a port 130 sized to receive the tip of a syringe to a lumen of the catheter 104. The base 206 and the hinged portion 122 may cooperate to clamp the distal end of the catheter 104.

In this example, the catheter 610 may extend through a first incision through the epidermis 602 and optionally through one or more layers of the dermis 604, through an abscess 606, and out from a second incision. The connector 106 includes the fluid port 130 to receive a sterile irrigation fluid, such as saline, medication, sterilized water, other fluids, or any combination thereof. The catheter 104 includes a lumen coupled to the irrigation port 130 and coupled to openings 116, which are positioned within the abscess 606, to deliver irrigation fluid into the abscess 606.

In the illustrated embodiment of FIG. 6, the catheter 104 and the connector 106 cooperate to facilitate the introduction of irrigation fluid via the irrigation port 130 and the openings 116. The connector 106 secures both ends of the catheter 104 to provide a secure loop that prevents the catheter 104 from being pulled out of the abscess. Further, the connector 106 prevents fluid flow through the portion of the catheter 104 that is clamped within the connector 106, making it possible to push irrigation fluid through a port in the connector 104 and through openings 116 into the abscess so that the fluid does not flow out the other end of the catheter 104. Other embodiments are also possible.

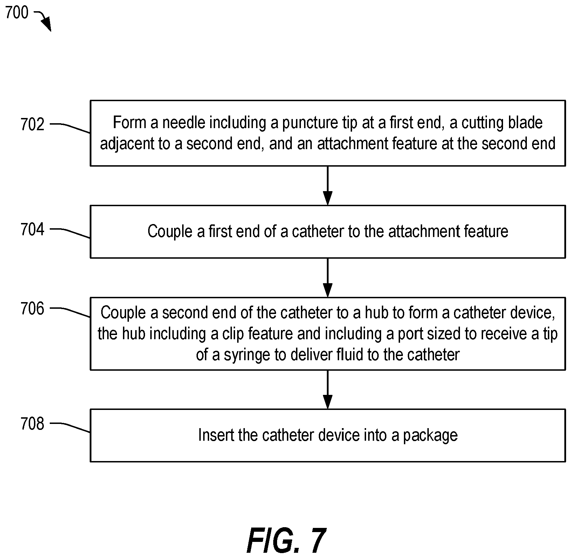

FIG. 7 depicts a flow diagram of a method 700 of producing a catheter device, in accordance with certain embodiments of the present disclosure. At 702, the method 700 can include forming a needle including a puncture tip at a first end, a cutting blade adjacent to a second end, and an attachment feature at the second end. Between the puncture tip and the cutting blade, the needle may include a curved body portion.

At 704, the method 700 can include coupling a first end of the catheter to the attachment feature. In some embodiments, the attachment feature may include a narrow portion and bulbous or wider portion. The attachment feature may be pressed into the lumen of the catheter, and the catheter may stretch to accommodate the bulbous or wider portion and may retract about the narrow portion to couple the catheter to the needle.

At 706, the method 700 can include coupling a second end of the catheter to a hub to form a catheter device, where the hub includes a clip feature and a port sized to receive a tip of a syringe to deliver fluid to the catheter. The catheter may fit over a catheter-engagement element, which may couple the catheter to the connector.

At 708, the method 700 may include inserting the catheter device into a package. The package may be a cardboard packaging including openings and securing features configured to secure the catheter device within the packaging. Other embodiments are also possible.

In conjunction with the devices described above with respect to FIGs. 1-7, a catheter device is disclosed that includes a needle, a catheter including a distal end coupled to the needle and including a proximal end, and a connector coupled to the proximal end. The connector may include a port sized to receive a tip of a syringe and may include a catheter-coupling element configured to couple the port to the lumen of the catheter to allow fluid to flow from the port into the lumen. The connector may further r include a base including a clip and a hinged element including a recess configured to engage the clip to clamp the hinged element to the base. The base may further include a recess configured to receive a distal portion of the catheter, and the hinged element may clamp the distal portion of the catheter to the base. Further, the hinged portion may include a post configured to compress the catheter to block fluid flow.

It should be appreciated that the catheter device described above may be used in a variety of contexts including but not limited to plastic surgery, breast surgery (to prevent collection of blood. lymph fluid, or both), orthopedic procedures, chest drainage, infected cysts, pancreatic surgery (to drain secretions), biliary surgery, thyroid surgery, neurosurgery (to remediate risk of intracranial pressure), urinary catheters, nasogastric tubes, and other procedures. In some embodiments, such as oral surgery, the size of the catheter device may be implemented with a smaller size in order to reduce irritation. In other embodiments, the hub may be larger in order to facilitate access to fluid ports. Other embodiments are also possible.

Although the present invention has been described with reference to preferred embodiments, workers skilled in the art will recognize that changes may be made in form and detail without departing from the scope of the invention, which is defined by the claims.

## Claims

1. A device comprising:
a catheter (104) including a proximal end and a distal end and defining a lumen extending from the proximal end to the distal end, the catheter including a plurality of openings (116) extending from the lumen through a wall of the catheter; and
a connector (106) coupled to the proximal end of the catheter and including a clamp configured to close over a portion of the catheter to compress the lumen to prevent fluid flow
a needle (102) including a connection element configured to couple the needle to the distal end of the catheter, wherein the needle includes a tip (110), a cutting blade (114), and a body portion (112) extending between the tip and the cutting blade,
wherein
the body portion (112) is curved to form an arcuate shape, and
the cutting blade (114) is substantially planar and comprises first and second sharpened edges (404, 406) that extend outward and away from the body portion (112),
**characterised in that**
the first sharpened edge is sharpened on a first side of the cutting blade but not a second side of the cutting blade and wherein the second sharpened edge is sharpened on the second side of the cutting blade but not the first side of the cutting blade.

2. The device of claim 1, wherein:
the clamp includes a base portion (206) including a recess (124) configured to receive the portion of the catheter;
a hinged portion (122) coupled to the base portion by a hinge (306), the hinged portion including a support defining an additional recess (208), wherein the base portion further includes a clip (120) configured to engage the additional recess to secure the hinged portion to the base portion in a closed state;
a post (128) extending from the hinged portion and configured to extend into the recess to compress the lumen when the hinged portion is closed over the catheter.

3. The device of claim 1, wherein the connector further includes a fluid port (130) configured to receive a tip of a syringe (108), the connector defining a fluid passage extending from the fluid port to the lumen of the catheter.

4. The device of claim 1, wherein the plurality of openings are closer to the proximal end than to the distal end of the catheter.

5. The device of claim 1 wherein:
the first sharpened edge is coupled to a first blunted edge (408),
the first blunted edge being proximal to the first sharpened edge;
the second sharpened edge is coupled to a second blunted edge (410),
the second blunted edge being proximal to the second sharpened edge.

6. The device of claim 5 wherein the connection element extends proximal to the first and second blunted edges.

7. The device of claim 6 wherein:
the needle includes a minimum breadth proximal to the tip;
the minimum breadth is included in the body portion proximal to the tip and distal to the first and second sharpened edges;
the minimum breadth does not abut either of the first or second sharpened edges;
the minimum breadth is measured orthogonal to a plane that intersects the tip, the cutting blade, and the body portion;
the plane intersects a void that extends between the tip and the body portion.

8. The device of claim 7 wherein the first and second sharpened edges taper outwardly away from the body portion as the first and second sharpened edges extend proximally and away from the tip.

9. The device of claim 8 wherein the tip, the cutting blade, the body portion, and the connection element are all monolithic with one another.

10. The device of claim 9 wherein at least a portion of the connection element is included within a distal portion of the lumen.

11. The device of claim 10 wherein:
the distal portion of the lumen is stretched around the portion of the connection element;
an addition portion of the lumen, which is proximal to the stretched distal portion of the lumen, is relaxed and unstretched.

## Patentansprüche

1. Vorrichtung, umfassend:
einen Katheter (104), der ein proximales Ende und ein distales Ende umfasst und ein Lumen definiert, das sich vom proximalen Ende zum distalen Ende erstreckt, wobei der Katheter eine Vielzahl von Öffnungen (116) umfasst, die sich vom Lumen durch eine Wand des Katheters erstrecken; und
einen Verbinder (106), der mit dem proximalen Ende des Katheters gekoppelt ist und eine Klemme umfasst, die so konfiguriert ist, dass sie sich über einem Abschnitt des Katheters schließt, um das Lumen zusammenzudrücken und einen Fluidfluss zu verhindern;
eine Nadel (102) mit einem Verbindungselement, das so konfiguriert ist, dass es die Nadel mit dem distalen Ende des Katheters koppelt, wobei die Nadel eine Spitze (110), eine Schneidklinge (114) und einen Körperabschnitt (112) aufweist, der sich zwischen der Spitze und der Schneidklinge erstreckt,
wobei
der Körperabschnitt (112) gekrümmt ist, um eine bogenförmige Form zu bilden, und
die Schneidklinge (114) im Wesentlichen eben ist und eine erste und eine zweite geschärfte Kante (404, 406) umfasst, die sich nach außen und von dem Körperabschnitt (112) weg erstrecken,
**dadurch gekennzeichnet, dass**
die erste geschärfte Kante an einer ersten Seite der Schneidklinge, aber nicht an einer zweiten Seite der Schneidklinge geschärft ist, und wobei die zweite geschärfte Kante an der zweiten Seite der Schneidklinge, aber nicht an der ersten Seite der Schneidklinge geschärft ist.

2. Vorrichtung nach Anspruch 1, wobei
die Klemme einen Basisabschnitt (206) mit einer Aussparung (124) umfasst, die so konfiguriert ist, dass sie den Abschnitt des Katheters aufnimmt;
einen Gelenkabschnitt (122), der mit dem Basisabschnitt durch ein Gelenk (306) gekoppelt ist, wobei der Gelenkabschnitt eine Stütze umfasst, die eine zusätzliche Aussparung (208) definiert, wobei der Basisabschnitt ferner eine Klammer (120) umfasst, die so konfiguriert ist, dass sie in die zusätzliche Aussparung eingreift, um den Gelenkabschnitt in einem geschlossenen Zustand an dem Basisabschnitt zu sichern;
ein Steg (128), der sich von dem Gelenkabschnitt erstreckt und so konfiguriert ist, dass er sich in die Aussparung erstreckt, um das Lumen zusammenzudrücken, wenn der Gelenkabschnitt über dem Katheter geschlossen wird.

3. Vorrichtung nach Anspruch 1, wobei der Verbinder ferner einen Fluidanschluss (130) umfasst, der so konfiguriert ist, dass er eine Spitze einer Spritze (108) aufnimmt, wobei der Verbinder einen Fluiddurchgang definiert, der sich von dem Fluidanschluss zu dem Lumen des Katheters erstreckt.

4. Vorrichtung nach Anspruch 1, wobei die Vielzahl von Öffnungen näher am proximalen Ende als am distalen Ende des Katheters liegt.

5. Vorrichtung nach Anspruch 1, wobei:
die erste geschärfte Kante mit einer ersten stumpfen Kante (408) gekoppelt ist, wobei die erste stumpfe Kante proximal zu der ersten geschärften Kante liegt;
die zweite geschärfte Kante mit einer zweiten stumpfen Kante (410) gekoppelt ist, wobei die zweite stumpfe Kante proximal zu der zweiten geschärften Kante liegt.

6. Vorrichtung nach Anspruch 5, wobei sich das Verbindungselement proximal zu der ersten und zweiten stumpfen Kante erstreckt.

7. Vorrichtung nach Anspruch 6, wobei:
die Nadel proximal zur Spitze eine Mindestbreite aufweist;
die Mindestbreite im Körperabschnitt proximal zur Spitze und distal zur ersten und zweiten geschärften Kante enthalten ist;
die Mindestbreite weder an die erste noch an die zweite geschärfte Kante angrenzt;
die Mindestbreite orthogonal zu einer Ebene gemessen wird, die die Spitze, die Schneidklinge und den Körperabschnitt schneidet;
die Ebene einen Hohlraum schneidet, der sich zwischen der Spitze und dem Körperabschnitt erstreckt.

8. Vorrichtung nach Anspruch 7, wobei sich die erste und zweite geschärfte Kante nach außen vom Körperabschnitt weg verjüngen, während sich die erste und zweite geschärfte Kante proximal und von der Spitze weg erstrecken.

9. Vorrichtung nach Anspruch 8, wobei die Spitze, die Schneidklinge, der Körperabschnitt und das Verbindungselement alle miteinander monolithisch sind.

10. Vorrichtung nach Anspruch 9, wobei mindestens ein Teil des Verbindungselements in einem distalen Teil des Lumens enthalten ist.

11. Vorrichtung nach Anspruch 10, wobei:
der distale Teil des Lumens um den Teil des Verbindungselements herum gestreckt ist;
ein zusätzlicher Teil des Lumens, der proximal zu dem gestreckten distalen Teil des Lumens liegt, entspannt und ungestreckt ist.

## Revendications

1. Dispositif comprenant :
un cathéter (104) comprenant une extrémité proximale et une extrémité distale et définissant une lumière s'étendant de l'extrémité proximale à l'extrémité distale, le cathéter comprenant une pluralité d'ouvertures (116) s'étendant à partir de la lumière à travers une paroi du cathéter ; et
un connecteur (106) couplé à l'extrémité proximale du cathéter et comprenant un dispositif de serrage configuré pour se fermer sur une partie du cathéter afin de comprimer la lumière pour empêcher l'écoulement de fluide,
une aiguille (102) comprenant un élément de raccordement configuré pour coupler l'aiguille à l'extrémité distale du cathéter, dans lequel l'aiguille comprend une pointe (110), une lame de coupe (114) et une partie de corps (112) s'étendant entre la pointe et la lame de coupe,
dans lequel :
la partie de corps (112) est courbée afin de former une forme arquée, et
la lame de coupe (114) est sensiblement plane et comprend des premier et second bords tranchants (404, 406) qui s'étendent vers l'extérieur et à l'opposé de la partie de corps (112),
**caractérisé en ce que** :
le premier bord tranchant est tranchant sur un premier côté de la lame de coupe, mais pas sur un second côté de la lame de coupe et dans lequel le second bord tranchant est tranchant sur le second côté de la lame de coupe, mais pas sur le premier côté de la lame de coupe.

2. Dispositif selon la revendication 1, dans lequel :
le dispositif de serrage comprend une partie de base (206) comprenant un évidement (124) configuré pour recevoir la partie du cathéter ;
une partie articulée (122) couplée à la partie de base par une charnière (306), la partie articulée comprenant un support définissant un évidement supplémentaire (208),
dans lequel la partie de base comprend en outre une attache (120) configurée pour mettre en prise l'évidement supplémentaire afin de fixer la partie articulée sur la partie de base dans un état fermé ;
un montant (128) s'étendant à partir de la partie articulée et configuré pour s'étendre dans l'évidement pour comprimer la lumière lorsque la partie articulée est fermée sur le cathéter.

3. Dispositif selon la revendication 1, dans lequel le connecteur comprend en outre un orifice de fluide (130) configuré pour recevoir une pointe d'une seringue (108), le connecteur définissant un passage de fluide s'étendant à partir de l'orifice de fluide à la lumière du cathéter.

4. Dispositif selon la revendication 1, dans lequel la pluralité d'ouvertures est plus proche de l'extrémité proximale que de l'extrémité distale du cathéter.

5. Dispositif selon la revendication 1, dans lequel :
le premier bord tranchant est couplé à un premier bord émoussé (408),
le premier bord émoussé étant proximal par rapport au premier bord tranchant ;
le second bord tranchant est couplé à un second bord émoussé (410), le second bord émoussé étant proximal par rapport au second bord tranchant.

6. Dispositif selon la revendication 5, dans lequel l'élément de raccordement s'étend de manière proximale par rapport aux premier et second bords émoussés.

7. Dispositif selon la revendication 6, dans lequel :
l'aiguille comprend une largeur minimum proximale par rapport à la pointe ;
la largeur minimum est incluse dans la partie de corps proximale par rapport à la pointe et distale par rapport aux premier et second bords tranchants ;
la largeur minimum ne vient pas en butée contre l'un ou l'autre des premier ou second bords tranchants ;
la largeur minimum est mesurée de manière orthogonale à un plan qui coupe la pointe, la lame de coupe et la partie de corps ;
le plan coupe un vide qui s'étend entre la pointe et la partie de corps.

8. Dispositif selon la revendication 7, dans lequel les premier et second bords tranchants se rétrécissent progressivement vers l'extérieur à l'opposé de la partie de corps, au fur et à mesure que les premier et second bords tranchants s'étendent de manière proximale et à l'opposé de la pointe.

9. Dispositif selon la revendication 8, dans lequel la pointe, la lame de coupe, la partie de corps et l'élément de raccordement sont tous monolithiques les uns avec les autres.

10. Dispositif selon la revendication 9, dans lequel au moins une partie de l'élément de raccordement est incluse dans une partie distale de la lumière.

11. Dispositif selon la revendication 10, dans lequel :
la partie distale de la lumière est étirée autour de la partie de l'élément de raccordement ;
une partie supplémentaire de la lumière, qui est proximale par rapport à la partie distale étirée de la lumière, est détendue et non étirée.
